# EUROPEAN PATENT APPLICATION

(11) **EP 3 032 217 A1**
(43) Date of publication of application: **15.06.2016**
(21) Application number: 14834390.8
(22) Date of filing: 01.07.2014
(51) Int. Cl.: G01B 11/24, A61B 1/00, G01B 11/16

(54) **OPTICAL SENSOR, OPTICAL SENSOR SYSTEM, AND ENDOSCOPE**

(30) Priority: 09.08.2013 JP 2013166882
(71) Applicant: Olympus Corporation, Tokyo 151-0072 (JP)
(72) Inventor: FUJITA, Hiromasa, Tokyo 151-0072 (JP); SATO, Ken, Tokyo 151-0072 (JP)
(74) Representative: Winter, Brandl, Fürniss, Hübner, Röss, Kaiser, Polte - Partnerschaft mbB
(86) International application number: PCT/JP2014/067538
(87) International publication number: WO 2015/019752

(57) **Abstract**

At least a part of the one characteristic change section 201 is arranged at a position different from at least a part of the other characteristic change section 203 in a circumferential direction of an optical sensor 10. At least a part of the one characteristic change section 201 is arranged at a position different from at least a part of the other characteristic change section 203 in an axial direction of an optical sensor 10.

## Description

### Technical Field

The present invention relates to an optical sensor, an optical sensor system having the optical sensor, and an endoscope having the optical sensor system.

### Background Art

For Example, Patent Literature 1 discloses an optical fiber that functions as an optical sensor. As shown in FIG. 8A, an optical fiber 401 has a core 401a that functions as a light guide member, a cladding 401b that covers the core 401a and functions as a light confinement member which confines light in the core 401a, and a light absorbing section 401c arranged in the cladding 401b. In addition, although not shown, the optical fiber 401 further has a protection member that covers the cladding 401b and protects the cladding 401b.

Light traveling through the optical fiber 401 will now be described.

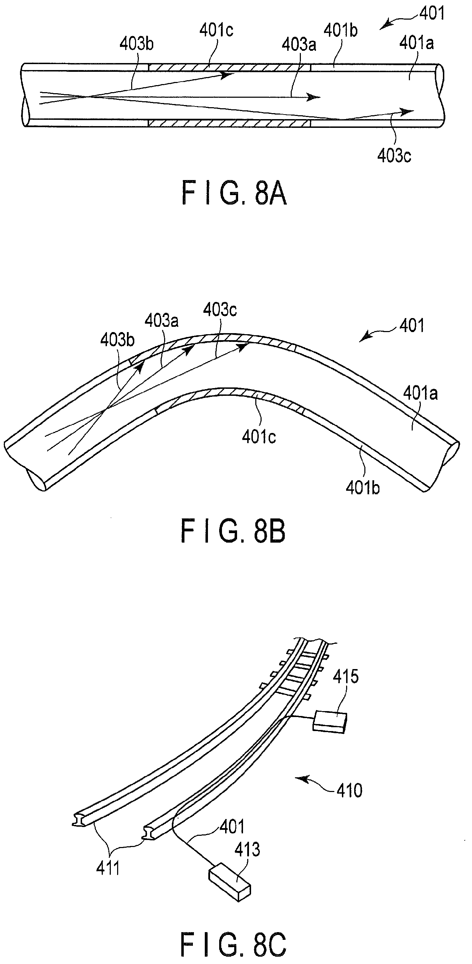

As shown in FIG. 8A, when the optical fiber 401 is straight, all light 403a traveling along an axial direction of the optical fiber 401 is guided. Light 403b traveling while inclined from the axial direction at a first angle is absorbed in the light absorbing section 401c. Light 403c traveling while inclined from the axial direction at a second angle is not absorbed in the light absorbing section 401c but totally reflected and guided by the cladding 401.

As shown in FIG. 8B, when the optical fiber 401 bends with the light absorbing section 401c at the center, the lights 403a, 403b, and 403c travel toward the light absorbing section 401c. Consequently, the lights 403a, 403b, and 403c are absorbed in the light absorbing section 401c, and do not travel. The light absorbing section 401c functions as a characteristics change section that changes optical characteristics of the light in accordance with a bending amount (a curvature) of the optical fiber 401.

An amount of the light guided in this way is displaced in accordance with a bending amount, and light intensity to be guided is controlled based on the displacement.

Such an optical fiber 401 is used in a curvature measuring apparatus 410 shown in FIG. 8C which is a typical example of an optical sensor system that detects a displacement of light intensity. The curvature measuring apparatus 410 shown in FIG. 8C has the optical fiber 401 which is shown in FIG. 8A and arranged along rails 411, a laser light source 413 optically connected to one end portion of the optical fiber 401, and a photoelectric conversion apparatus 415 optically connected to the other end portion of the optical fiber 401. The optical fiber 401 bends in accordance with bending of the rails 411. With this bending, an amount of light traveling from the laser light source 413 to the photoelectric conversion apparatus 415 through the optical fiber 401 is reduced. Furthermore, the photoelectric conversion apparatus 415 measures an amount of the light which is reduced in accordance with the bending. Consequently, a bending amount of the rails 411 and a level of sinking of the rails 411 when a train passes are measured.

### Citation List

### Patent Literature

Patent Literature 1: Jpn. Pat. Appln. KOKAI Publication No. 57-141604

### Summary of Invention

### Technical Problem

Such a curvature measuring apparatus 410 as shown in FIG. 8C only detects a bending amount of the optical fiber 401 by using one optical fiber 401, and it cannot detect a bending direction.

Further, the curvature measuring apparatus 410 cannot detect two axes, e.g., a bending amount of the optical fiber 401 in up and down directions of the optical fiber 401 and a bending amount of the optical fiber 401 in left and right directions of the optical fiber 401 by using one optical fiber 401.

That is, two axes such as two bending amounts different from each other or two axes such as two bending directions different from each other cannot be detected.

Furthermore, to detect two axes by the one optical fiber 401, the two light absorbing sections 401c must be arranged in the one optical fiber 401. In this case, one light absorbing section 401c must be apart from the other light absorbing section 401c at, e.g., 90 degrees in a circumferential direction of the optical fiber 401. In such two light absorbing sections 401c, when the one light absorbing section 401c and the other light absorbing section 401c are arranged on the same circumference, it is not easy to process the two light absorbing sections 401c so that a strength of the optical fiber 401 can be assured.

Furthermore, usually, an inner peripheral surface of the protection member does not adhere to an outer peripheral surface of the cladding 401b. Thus, when the optical fiber 401 bends, there is a fear that a part of the protection member placed between the two light absorbing sections 401c in the circumferential direction of the optical fiber 401 shifts from the cladding 401b in, e.g., the circumferential direction of the optical fiber 401, and there is also a fear that this member comes off.

The present invention has been developed in view of these circumstances, and it is an object of the present invention to provide an optical sensor that can easily detect two axes by one optical sensor, has a characteristic change section that can be easily processed to assure the strength of the optical sensor, and can suppress a shifting and coming-off of a protection member from a light confinement member, an optical sensor system having this optical sensor, and an endoscope having this optical sensor system.

### Solution to Problem

An aspect of an optical sensor of the present invention including; a light guide member which guides light; a light confinement member which covers an outer peripheral surface of the light guide member to abut on the outer peripheral surface of the light guide member, and confines the light in the light guide member; a protection member which covers an outer peripheral surface of the light confinement member to abut on the outer peripheral surface of the light confinement member, and protects the light confinement member; and characteristic change sections which are in contact with at least the light guide member, and change optical characteristics of the light in accordance with a bending amount of the light guide member, wherein one characteristic change section and the other characteristic change section are arranged to be close to each other in an area having a desired range, at least a part of the one characteristic change section is arranged at a position different from at least a part of the other characteristic change section in a circumferential direction of the optical sensor, at least a part of the one characteristic change section is arranged at a position different from at least a part of the other characteristic change section in an axial direction of the optical sensor, the one characteristic change section changes the optical characteristics in accordance with the bending amount in one direction of the light guide member in the area, the other characteristic change section changes the optical characteristics in accordance with the bending amount in the other direction of the light guide member in the area, and the characteristic change sections change the optical characteristics so that the one optical characteristics changed by the one characteristic change section are different from the other optical characteristics changed by the other characteristic change section, when the optical characteristics of the light change.

An aspect of an optical sensor system of the present invention including: a light source which emits the light toward the light guide member; the optical sensor; and a detecting section which independently detects the light having the optical characteristics on the one side and the light having the optical characteristics on the other side, and detects mutually different bending directions each other and mutually different bending amounts each other in the optical sensor based on a detection result.

An aspect of an endoscope of the present invention including the optical sensor system.

### Brief Description of Drawings

FIG. 1 is a perspective view of an optical sensor according to a first embodiment of the present invention, a cross-sectional view taken along 1A-1A shown in this perspective view, a cross-sectional view taken along 1B-1B shown in this perspective view, and a cross-sectional view taken along 1C-1C shown in this perspective view;
FIG. 2A is a perspective view for detection of two axes;
FIG. 2B is a conceptual view showing that optical characteristics are optical absorption characteristics;
FIG. 2C is a conceptual view showing that the optical characteristics are wavelength conversion characteristics;
FIG. 3A is a schematic view of an optical sensor system having the optical sensor shown in FIG. 1A;
FIG. 3B is a schematic view of an endoscope having the optical sensor system shown in FIG. 3A;
FIG. 4A is a schematic view of an optical sensor different from the optical sensor according to the first embodiment, and a cross-sectional view taken along 4A-4A shown in this perspective view;
FIG. 4B is a schematic view of a light transmission amount according to bending of the optical sensor;
FIG. 4C is a schematic view of a light transmission amount according to bending of the optical sensor;
FIG. 4D is a schematic view of a light transmission amount according to bending of the optical sensor;
FIG. 4E is a view showing a relationship between a bending amount of the optical sensor and a change rate of optical characteristics;
FIG. 5A is a view showing an example of an arrangement position of a characteristic change section;
FIG. 5B is a view showing an example of the arrangement position of the characteristic change section;
FIG. 5C is a view showing an example of the arrangement position of the characteristic change section;
FIG. 5D is a view showing an example of the arrangement position of the characteristic change section;
FIG. 5E is a view for explaining an inflow preventing section;
FIG. 5F is a view showing FIG. 5E from an arrow 5F depicted in FIG. 5E;
FIG. 6A is a view showing that the optical sensor has areas;
FIG. 6B shows a modification of the optical sensor system;
FIG. 7A is a view showing a first modification of the optical sensor;
FIG. 7B is a view showing a second modification of the optical sensor;
FIG. 7C is a view showing a third modification of the optical sensor;
FIG. 7D is a view showing a fourth modification of the optical sensor;
FIG. 7E is a view showing a fifth modification of the optical sensor;
FIG. 8A is a view showing a linear optical fiber that functions as a general optical sensor;
FIG. 8B is a view showing a state that the optical fiber depicted in FIG. 8A is bent; and
FIG. 8C is a view showing a curvature measuring apparatus which is an optical sensor system having the optical fiber depicted in FIG. 8A.

### Brief Description of Embodiments

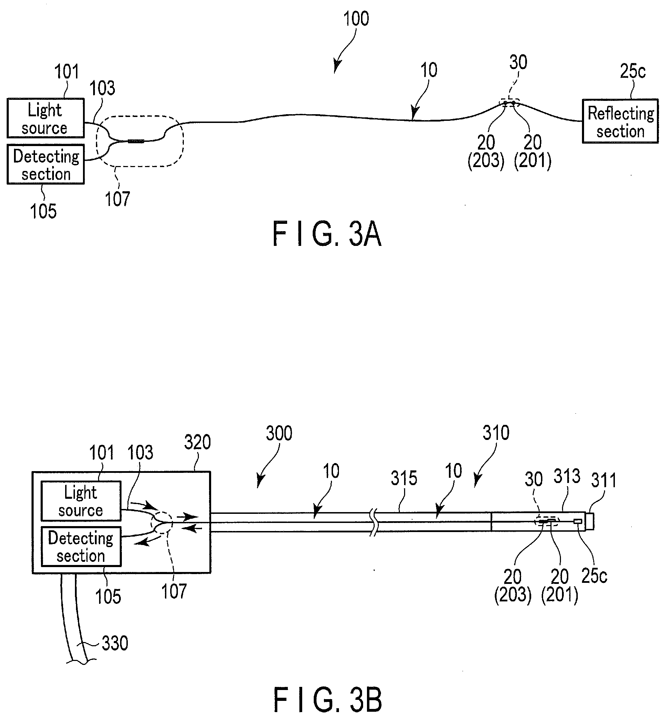

An embodiment according to the present invention will now be described hereinafter with reference to the drawings. It is to be noted that, for example, as with omission of a light guide member 11 in FIG. 3A, some members are omitted in some of the drawings for clarification of diagrammatic representation.

### [First Embodiment]

### [Configuration]

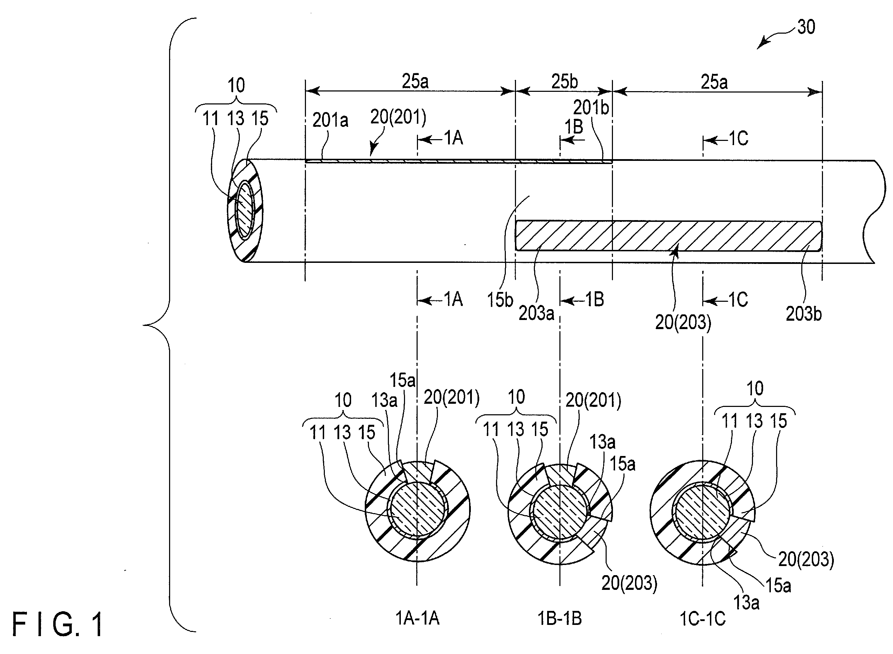

A first embodiment will now be described with reference to FIG. 1, FIG. 2A, FIG. 2B, FIG. 2C, FIG. 3A, FIG. 3B, FIG. 4A, FIG. 4B, FIG. 4C, FIG. 4D, and FIG. 4E.

### [Optical Sensor 10]

An optical sensor 10 shown in FIG. 1 has, e. g., flexibility. The optical sensor 10 has optical members, e.g., an optical fiber, a waveguide, and others. The waveguide may have a configuration in which thin films are laminated in several layers.

As shown in FIG. 1, the optical sensor 10 has a light guide member 11 which guides light, and a light confinement member 13 which covers an outer peripheral surface of the light guide member 11 to abut on the outer peripheral surface of the light guide member 11 and confines the light in the light guide member 11. Moreover, the optical sensor 10 further has a protection member 15 which covers an outer peripheral surface of the light confinement member 13 to abut on the outer peripheral surface of the light confinement member 13 and protects the light confinement member 13.

### [Light Guide Member 11/Light Confinement Member 13/Protection Member 15]

As shown in FIG. 1, the light guide member 11 functions as a nucleus of the optical sensor 10. The light guide member 11 has a core. The light guide member 11 has, e.g., a pillar shape, specifically a columnar shape.

The light confinement member 13 as shown in FIG. 1 has a cladding. The light confinement member 13 has, e.g. , a tubular shape, specifically a cylindrical shape.

The protection member 15 as shown in FIG. 1 has a jacket. The protection member 15 has, e.g., a tubular shape, specifically a cylindrical shape. The protection member 15 and the light confinement member 13 function as cover members that cover the light guide member 11.

As shown in FIG. 1, the light confinement member 13 has a notch portion 13a formed by notching a part of the light confinement member 13 so that a part of the outer peripheral surface of the light guide member 11 is exposed.

Like the above, the protection member 15 has a notch portion 15a which is arranged on the same straight line as the notch portion 13a in a radial direction of the optical sensor 10, communicates with the notch portion 13a in the radial direction of the optical system 10, and is formed by notching a part of the protection member 15.

Each of the notch portion 13a and the notch portion 15a is arranged at multiple positions, e.g., two positions. Since the arrangement positions of the notch portion 13a and the notch portion 15a correspond to arrangement positions of later-described characteristic change sections 201 and 203, a detailed description will be given later.

### [Characteristic Change Sections 20]

Moreover, as shown in FIG. 1, the optical sensor 10 further has characteristic change sections 20 that are in contact with at least the light guide member 11 and change optical characteristics of the light guided by the light guide member 11 in accordance with a bending amount of the light guide member 11.

As shown in FIG. 1, the characteristic change sections 20 are arranged in the notch portions 13a and 15a to contact the outer peripheral surface of the light guide member 11, and embedded in the notch portions 13a and 15a. An outer peripheral surface of each characteristic change section 20 does not protrude to the outer peripheral surface of the protection member 15 in the radial direction of the optical sensor 10, and is arranged on substantially the same plane as the outer peripheral surface of the protection member 15. Additionally, side surfaces of each characteristic change section 20 are in contact with side surfaces of the light confinement member 13 and side surfaces of the protection member 15. An inner peripheral surface of each characteristic change section 20 is in contact with the outer peripheral surface of the light guide member 11.

It is to be noted that each characteristic change section 20 does not have to be arranged in each overall notch portion 13a or 15a; it may be formed in accordance with a hardness of the characteristic change section 20 and a thickness of the characteristic change section 20. In this case, the characteristic change section 20 has a thickness according to a light response of the characteristic change section 20.

For example, the following description will be given on the assumption that two characteristic change sections 201 and 203 are arranged.

As shown in FIG. 1, a center axis of one characteristic change section 201 and a center axis of the other characteristic change section 203 are arranged along an axial direction of the optical sensor 10. In more detail, the characteristic change section 201 and the characteristic change section 203 are arranged along a path of the light propagated through the light guide member 11. The characteristic change section 201 has the same size and the same shape as the characteristic change section 203. The characteristic change sections 201 and 203 have, e.g., a rectangular shape.

As shown in FIG. 1, the characteristic change sections 201 and 203 are arranged to be close to each other in one area 30 having a desired range. This area 30 represents one desired narrow area 30 such as a distal end portion of the optical sensor 10, for example.

### [Arrangement Positions of Characteristic Change Sections 201 and 203 in Circumferential Direction of Optical Sensor 10]

As shown in FIG. 1, one overall characteristic change section 201 is arranged at a position different from the other overall characteristic change section 203 in a circumferential direction of the optical sensor 10. In other words, the characteristic change section 201 is arranged to shift from the characteristic change section 203 in the circumferential direction of the optical sensor 10. The circumferential direction of the optical sensor 10 is a direction which is along to the outer periphery of the light guide member 11 in a cross section of the light guide member 11 which is a cross section (for example, vertically) cutting across a propagation path of the light.

As shown in FIG. 1, the characteristic change section 201 is arranged to be apart from the characteristic change section 203 at, e.g., 120 degrees in the circumferential direction of the optical sensor 10. It is to be noted that the characteristic change section 201 may be arranged to be apart from the characteristic change section 203 at, e.g., 90 degrees in the circumferential direction of the optical sensor 10. In the axial direction of the optical sensor 10, the characteristic change section 201 is not coaxially arranged to the characteristic change section 203.

### [Arrangement Positions of Characteristic Change Sections 201 and 203 in Axial Direction of Optical Sensor 10]

Further, as shown in FIG. 1, the characteristic change section 201 is arranged at a position different from the characteristic change section 203 in the axial direction of the optical sensor 10. In other words, the characteristic change section 201 is arranged to shift from the characteristic change section 203 in the axial direction of the optical sensor 10.

As shown in the perspective view in FIG. 1, a distal end portion 201a of the characteristic change section 201 is arranged to be closer to the distal end of the optical sensor 10 than a distal end portion 203a of the characteristic change section 203 in the axial direction of the optical sensor 10. Furthermore, as shown in the perspective view in FIG. 1, the proximal end portion 201b of the characteristic change section 201 is arranged to be closer to the distal end of the optical sensor 10 than a proximal end portion 203b of the characteristic change section 203 in the axial direction of the optical sensor 10. As described above, as shown in a cross-sectional view taken along 1A-1A and a cross-sectional view taken along 1C-1C in FIG. 1, a part of the characteristic change section 201 is arranged not to be superimposed on a part of the characteristic change section 203 in the circumferential direction of the optical sensor 10; in other words, arranged not to be on the same circumference. This part represents a combination of the distal end portion 201a and the distal end portion 203a or a combination of the proximal end portion 201b and the proximal end portion 203b.

Specifically, as shown in the perspective view in FIG. 1 and the cross-sectional view taken along 1A-1A in FIG. 1, the distal end portion 201a of the characteristic change section 201 is arranged not to be superimposed on the distal end portion 203a of the characteristic change section 203 in the circumferential direction of the optical sensor 10. The non-superimposition includes non-overlapping. In other words, the distal end portion 201a of the characteristic change section 201 is arranged not to be on the same circumference of the optical sensor 10 to the distal end portion 203a of the characteristic change section 203. As described above, the distal end portion 201a is arranged at a position different from the distal end portion 203a in the axial direction of the optical sensor 10 and the circumferential direction of the optical sensor 10.

Moreover, as shown in the perspective view in FIG. 1 and the cross-sectional view taken along 1B-1B in FIG. 1, the proximal end portion 201b of the characteristic change section 201 is arranged to be superimposed on the distal end portion 203a of the characteristic change section 203 in the circumferential direction of the optical sensor 10. The superimposition includes overlapping. In other words, the proximal end portion 201b of the characteristic change section 201 is arranged on the same circumference of the optical sensor 10 to the distal end portion 203a of the characteristic change section 203 and aligned with the same. As described above, the proximal end portion 201b is arranged at the same position to the distal end portion 203a in the axial direction of the optical sensor 10, but arranged at a position different from the distal end portion 203a in the circumferential direction of the optical sensor 10.

Additionally, as shown in the perspective view in FIG. 1 and the cross-sectional view taken along 1C-1C in FIG. 1, the proximal end portion 201b of the characteristic change section 201 is arranged not to be superimposed on the proximal end portion 203b of the characteristic change section 203 in the circumferential direction of the optical sensor 10. The non-superimposition includes non-overlapping. In other words, the proximal end portion 201b of the characteristic change section 201 is arranged not to be on the same circumference of the optical sensor 10 to the proximal end portion 203b of the characteristic change section 203. As described above, the proximal end portion 201b is arranged at a position different from the proximal end portion 203b in the axial direction of the optical sensor 10 and the circumferential direction of the optical sensor 10.

To sum up, the characteristic change section 201 has the distal end portion 201a that functions as a non-superimposing portion 25a that is not superimposed on the characteristic change section 203 in the circumferential direction of the optical sensor 10, and the proximal end portion 201b that functions as a superimposing portion 25b that is superimposed on the characteristic change section 203 in the circumferential direction of the optical sensor 10.

Further, the characteristic change section 203 has the distal end portion 203a that functions as the superimposing portion 25b that is superimposed on the characteristic change section 201 in the circumferential direction of the optical sensor 10, and the proximal end portion 203b that functions as the non-superimposing portion 25a that is not superimposed on the characteristic change section 201 in the circumferential direction of the optical sensor 10.

Furthermore, the optical sensor 10 has the superimposing portion 25b and the non-superimposing portions 25a that sandwich the superimposing portion 25b. The superimposing portion 25b and the non-superimposing portions 25a are arranged along the axial direction of the optical sensor 10.

As described above, in the characteristic change sections 201 and 203 arranged at positions different from each other in the circumferential direction of the optical sensor 10, a part (the proximal end portion 201b) of the characteristic change section 201 is superimposed on a part (the distal end portion 203a) of the characteristic change section 203 in the circumferential direction of the optical sensor 10. Moreover, in the characteristic change sections 201 and 203 arranged at positions different from each other in the circumferential direction of the optical sensor 10, a part (e.g., the distal end portion 201a) of the characteristic change section 201 is arranged at a position different from a part (e.g. , the distal end portion 203a) of the characteristic change section 203 in the axial direction of the optical sensor 10.

It is to be noted that, in this embodiment, the notch portions 13a and 15a are arranged in accordance with the positions of the characteristic change sections 201 and 203.

### [Bending Amounts and Bending Directions Based on Optical Characteristics of Characteristic Change Sections 201 and 203]

Based on the arrangement of the characteristic change sections 201 and 203, the characteristic change section 201 changes optical characteristics in accordance with a bending amount in one direction, e.g. , an up-and-down direction of the light guide member 11 in the area 30. Further, the characteristic change section 203 changes the optical characteristics in accordance with a bending amount in the other direction, e.g. , a left-and-right direction of the light guide member 11 in the area 30. The light guide member 11 in the area 30 represents a part of the light guide member 11 where the area 30 having the characteristic change section 201 and 203 is arranged rather than bending of the entire light guide member 11. It is to be noted that, in the optical characteristics changed by the characteristic change section 201, a variation amount in the optical characteristics changes in accordance with a bending amount of the light guide member 11. This is also true to the characteristic change section 203.

As described above, a change in the optical characteristics of the characteristic change section 201 is independent of a change in the optical characteristics of the characteristic change section 203.

Furthermore, the above configuration enables detecting two axes, e.g., a bending amount of the optical sensor 10 in the up-and-down direction of the optical sensor 10 and a bending amount of the optical sensor 10 in the left-and-right direction of the optical sensor 10.

In other words, when the characteristic change section 201 or the characteristic change section 203 changes the optical characteristics, a bending direction of the optical sensor 10 and a bending amount in this bending direction can be detected.

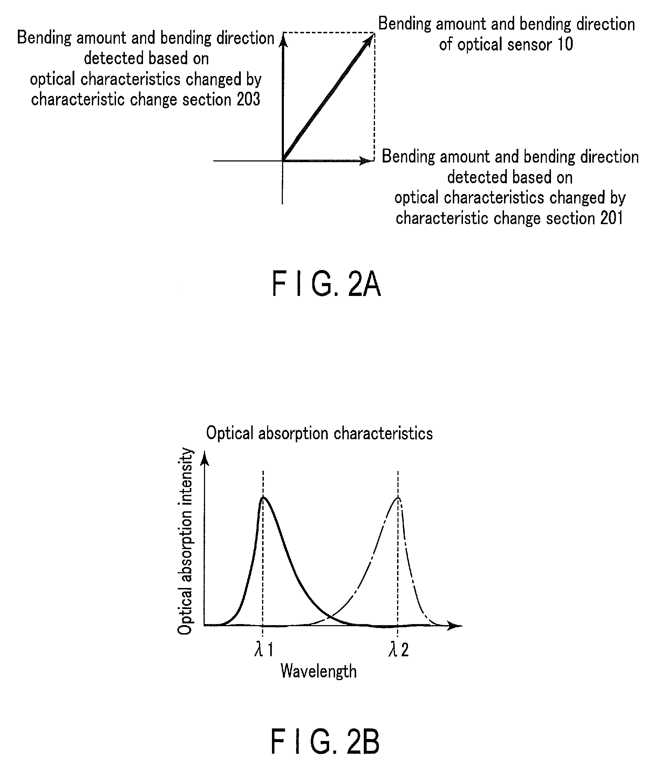

Moreover, as shown in FIG. 2A, as a ratio of the bending direction detected based on the optical characteristic changed by the characteristic change section 201 and the bending direction detected based on the optical characteristics changed by the characteristic change section 203 is synthetized, the bending direction of the optical sensor 10 itself can be detected. Additionally, when the ratio of the bending amount detected based on the optical characteristics changed by the characteristic change section 201 and the bending amount detected based on the optical characteristics changed by the characteristic change section 203 is synthetized, the bending amount in the bending amount of the optical sensor 10 itself can be detected.

### [Optical Characteristics of Characteristic Change Section 201 and 203]

When the optical characteristics of the light are changed by the characteristic change sections 20, the characteristic change sections 201 and 203 change the optical characteristics so that optical characteristics A as one changed by the characteristic change section 201 become different from optical characteristics B as the other changed by the characteristic change section 203. That is, the optical characteristics in the characteristic change section 201 are different from the optical characteristics in the characteristic change section 203. The optical characteristics in the characteristic change section 201 are independent of the optical characteristics in the characteristic change section 203.

Thus, the characteristic change section 201 is made of a material A having the optical characteristics A, and the characteristic change section 203 is made of a material B having the optical characteristics B different from the optical characteristic A of the material A.

As an example of the optical characteristics, there are optical absorption characteristics of absorbing light having a specific wavelength as shown in FIG. 2B.

The material A of the characteristic change section 201 has a pigment A that absorbs light having a wavelength λ1 in the light propagated through the light guide member 11, and functions as a light absorbing section. Additionally, the material B of the characteristic change section 203 has a pigment B that absorbs light having a wavelength λ2 different from the wavelength λ1 in the light propagated through the light guide member 11, and functions as a light absorbing section.

In this case, each of the characteristic change sections 201 and 203 is formed as a soft member having substantially the same refraction index as that of the light confinement member 13 having a pigment mixed therein or a soft member having substantially the same refraction index as the light guide member 11. The latter soft member may contain, e.g., glass. In this embodiment, the characteristic change sections 201 and 203 mainly absorb the wavelengths λ1 and λ2 in evanescent light to the light confinement member 13.

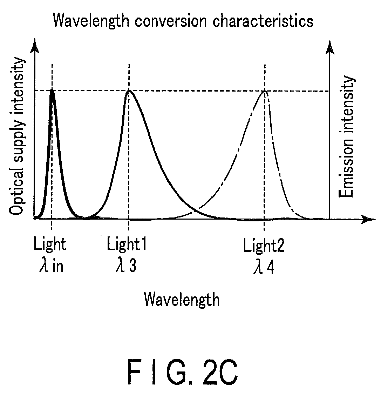

Additionally, as an example of the optical characteristics, there are, e. g. , wavelength conversion characteristics as shown in FIG. 2C.

The characteristic change section 201 has a member A that absorbs light having a wavelength λin and emits light 1 having a wavelength λ3 different from this wavelength λin of the light. Further, the characteristic change section 203 has a member B that absorbs light having the wavelength λin and emits light 2 having a wavelength λ4 different from the wavelength λin and the wavelength λ3 of the light. These members A and B have, e.g., a fluorescent substance, for example.

As a matter of course, the example of the optical characteristic is not restricted to the above.

### [Optical Sensor System 100 having Optical Sensor 10]

As shown in FIG. 3A, the optical sensor 10 is mounted in an optical sensor system 100.

This optical sensor system 100 has a light source 101 that emits light toward the light guide member 11, a supply light guide member 103 that guides the light emitted from the light source 101 to supply the light to the optical sensor 10, and the optical sensor 10 which is a characteristic light guide member that further guides the light guided by the supply light guide member 103.

Further, the optical sensor system 100 further has a detecting section 105 that detects light whose optical characteristics are changed by the characteristic change sections 20 arranged in the optical sensor 10 and which is guided by the optical sensor 10.

Furthermore, the optical sensor system 100 further has an optical branch section 107 which is optically connected to the supply light guide member 103, the optical sensor 10, and the detecting section 105, and branches the light so that the light can be guided to the optical sensor 10 from the supply light guide member 103 and the light is guided to the detecting section 105 from the optical sensor 10.

### [Light Source 101]

The light source 101 is optically connected to the supply light guide member 103. The light emitted from the light source 101 enters the supply light guide member 103. The light source 101 has, e.g., a laser light source that emits a laser beam, an LED light source that emits LED light, a lamp light source that emits lamp light, a fluorescent material that emits fluorescent light, or a combination of these members.

It is to be noted that the light emitted from the light source 101 may be focused by an optical member such as a convex lens, thereby enter the supply light guide member 103. Consequently, efficiency of the light that enters the supply light guide member 103 is improved. The light source 101 may independently emit light corresponding to the characteristic change section 201 and light corresponding to the characteristic change section 203, or may emit light corresponding to the characteristic change section 201 or 203 alone.

Moreover, the light source 101 may be directly optically connected to the optical branch section 107 without arranging the supply light guide member 103.

In this state, if the optical branch section 107 has, e.g., a fiber coupler, the light source 101 may have a lens system that focuses light to the optical fiber of the fiber coupler.

In this state, if the optical branch section 107 has, e.g. , a half mirror or a beam splitter, the light source 101 may have a lens system that converts the light into parallel light.

Additionally, when return light affects an output like a laser diode, the light source 101 may include an isolator or the like.

### [Supply Light Guide Member 103]

The supply light guide member 103 has one end portion optically connected to the light source 101 and the other end portion optically connected to the optical branch section 107. The supply light guide member 103 has, e.g., flexibility. The supply light guide member 103 has, e.g., an optical fiber.

### [Optical Sensor 10]

The optical sensor 10 has one end portion optically connected to the optical branch section 107 and the other end portion having a reflecting section 25c. The reflecting section 25c reflects light guided from the one end portion toward the one end portion. The reflecting section 25c has a mirror formed by vapor-depositing aluminum or the like on the other end portion. Thus, in this embodiment, the light travels from the light source 101 to the detecting section 105, and is turned back at the reflecting section 25c and its periphery including the characteristic change sections 20 as a relay point.

### [Detecting Section 105]

The detecting section 105 independently detects the light having the optical characteristics A changed by the characteristic change section 201 and the light having the optical characteristics B changed by the characteristic change section 203. Further, the detecting section 105 detects different bending directions each other and different bending amounts each other in the optical sensor 10 based on a detection result.

To independently detect the optical characteristics, the multiple detecting sections 105 may be arranged in accordance with, e.g. , the characteristic change sections 201 and 203. The detecting section 105 has, e.g., a spectroscopic sensor or a spectroscope.

### [Optical Branch Section 107]

The optical branch section 107 has one end portion which is bifurcated and the other end portion. One of the one end portion is optically connected with the other end portion of the supply light guide member 103, the other end portion is optically connected with one end portion of the optical sensor 10, and the other of the one end portion is optically connected with the detecting section 105. Consequently, the optical branch section 107 guides the light guided by the supply light guide member 103 to the optical sensor 10, and guides the light guided by the optical sensor 10 to the detecting section 105. The optical branch section 107 prevents the light guided by the supply light guide member 103 from traveling to the detecting section 105, and also prevents the light guided by the optical sensor 10 from returning to the supply light guide member 103. The optical branch section 107 is formed as a light guide path. The optical branch section 107 is formed of, e.g. , a film with a low refraction index and films with a high refraction index that sandwich the film with a low refraction index.

The light source 101 and the detecting section 105 are arranged on the one end portion side of the optical branch section 107, and the optical sensor 10 including the characteristic change sections 20 is arranged on the other end portion side of the optical branch section 107. Furthermore, the optical sensor system 100 is arranged in such a manner that the light travels from the light source 101 to the detecting section 105, and is turned back at the reflecting section 25c and its periphery including the characteristic change sections 20 as a relay point.

### [Endoscope 300 having Optical Sensor System 100]

As shown in FIG. 3B, the optical sensor system 100 is mounted in an endoscope 300, and arranged in the endoscope 300.

This endoscope 300 has a hollow elongated inserting section 310 that is inserted into, e. g. , a lumen in a body cavity, an operating section 320 that is coupled with a proximal end portion of the inserting section 310 and operates the endoscope 300, and a universal cord 330 that is connected with the operating section 320 and extended from a side surface of the operating section 320.

The inserting section 310 has a distal rigid section 311, a bending section 313, and a flexible tube section 315 from a distal end portion side of the inserting section 310 toward the proximal end portion side of the inserting section 310. A proximal end portion of the distal rigid section 311 is coupled with a distal end portion of the bending section 313, and a proximal end portion of the bending section 313 is coupled with a distal end portion of the flexible tube section 315. The bending section 313 bends in desired directions, e.g., up, down, left, and right directions by an operation of the operating section 320.

For example, the light source 101, the supply light guide member 103, the optical branch section 107, and the detecting section 105 are arranged in the operating section 320, and the optical sensor 10 is arranged in the operating section 320 and in the inserting section 310. Furthermore, the characteristic change sections 201 and 203 are positioned to be arranged in, e.g., the bending section 313.

Consequently, the optical sensor system 100 detects bending directions of the bending section 313 and bending amounts of the bending section 313 based on a detection result detected by the detecting section 105.

Moreover, the endoscope 300 is included in an endoscope apparatus arranged in, e.g. , a laboratory or an operating room. This endoscope apparatus has the endoscope 300 and an image processing device (e.g., a video processor) that executes image processing to an image of a body cavity of a patient or the like acquired by the endoscope 300. Additionally, the endoscope apparatus further has a display section that is connected with the image processing device and displays an image of a body cavity of a patient or the like that is acquired by the endoscope 300 and subjected to image processing by the image processing device, and a light source device that emits light for illumination light emitted from the endoscope 300. In addition, the endoscope apparatus further has a control device that controls the entire endoscope apparatus including the endoscope 300, the image processing device, the display device, and the light source device.

The universal cord 330 has a connection connector that can be attached to or detached from the image processing device and the light source device. The connection connector is arranged to connect the endoscope 300 with various kinds of devices (the image processing device, the light source device), and transmit or receive data between these members.

The image processing device, the light source device, and the control device are electrically connected to each other. The image processing device and the light source device are detachably connected to the endoscope 300 through the connection connector.

The display unit may show bending directions of the bending section 313 and bending amounts of the bending section 313 detected by the optical sensor system 100.

It is to be noted that the connection connector may have a feedback system that stabilizes an operation of the light source 101 mounted therein.

Additionally, the optical sensor 10 may be arranged in the universal cord 330. Further, the characteristic change sections 201 and 203 are positioned to be arranged in, e.g. , the universal cord 330.

Consequently, the optical sensor system 100 detects bending directions of the universal cord 330 and bending amounts of the universal cord 330 based on a detection result of detection effected by the detecting section 105.

Furthermore, the light source device may function as the light source 101, the supply light guide member 103 may be arranged in the universal cord 330, the optical branch section 107 maybe arranged in the connection connector, and the control device may function as the detecting section 105.

### [Operation]

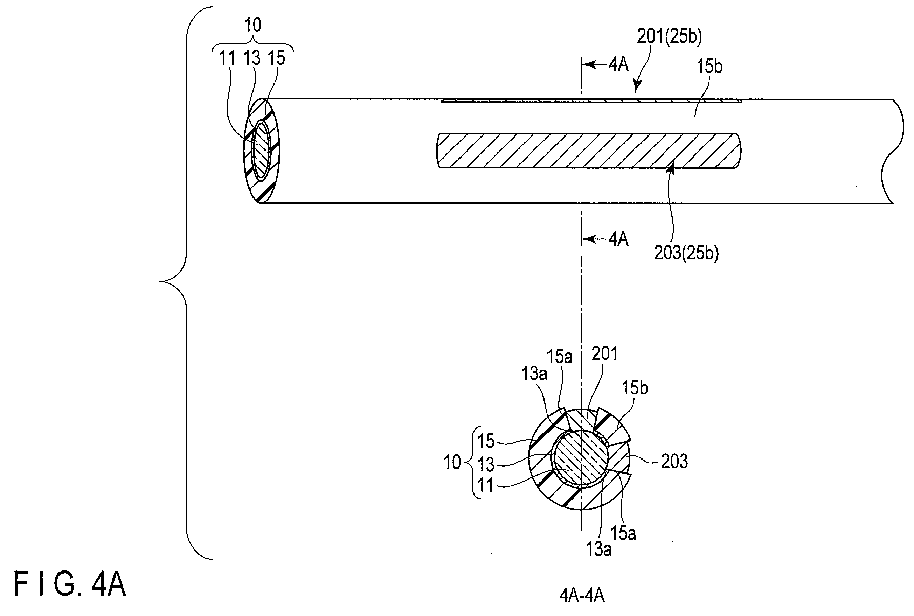

As shown in FIG. 4A, like this embodiment, for example, it is assumed that the characteristic change section 201 is arranged to shift from the characteristic change section 203 in the circumferential direction of the optical sensor 10. Moreover, differing from this embodiment, as shown in FIG. 4A, the overall characteristic change section 201 is arranged at the same position as the overall characteristic change section 203 in the axial direction of the optical sensor 10. That is, the characteristic change section 201 is assumed to be arranged on the same circumference to the characteristic change section 203. In other words, the overall characteristic change section 201 and the overall characteristic change section 203 are assumed to function as the superimposing portions 25b.

In this case, the overall characteristic change section 201 including the notch portions 13a and 15a is close to the characteristic change section 203 including the notch portions 13a and 15a in the circumferential direction of the optical sensor 10. Thus, it is not easy to process the notch portions 13a and 15a so that a strength of the optical sensor 10 in portions where these members are arranged can be assured, and also not easy to arrange the characteristic change sections 201 and 203 in the notch portions 13a and 15a, respectively.

Additionally, usually, the inner peripheral surface of the protection member 15 does not adhere to the outer peripheral surface of the light confinement member 13. Thus, when the optical sensor 10 bends, a part 15b of the protection member 15 placed between the characteristic change sections 201 and 203 in the circumferential direction of the optical sensor 10 may possibly shift from the light confinement member 13 in, e. g. , the circumferential direction of the optical sensor 10, and may possibly come off.

However, as shown in FIG. 1, in this embodiment, the characteristic changes section 201 including the notch portions 13a and 15a is arranged to shift from the characteristic change section 203 including the notch portions 13a and 15a in the circumferential direction of the optical sensor 10. Further, the characteristic change section 201 including the notch portions 13a and 15a is arranged at a position different from the characteristic change section 203 including the notch portions 13a and 15a in the axial direction of the optical sensor 10. Specifically, the distal end portion 201a of the characteristic change section 201 is arranged to be closer to the distal end of the optical sensor 10 than the distal end portion 203a of the characteristic change section 203. That is, the distal end portion 201a of the characteristic change section 201 and the proximal end portion 203b of the characteristic change section 203 function as the non-superimposing portions 25a, and the proximal end portion 201b of the characteristic change section 201 and the distal end portion 203a of the characteristic change section 203 function as the superimposing portion 25b.

Thus, the overall characteristic change section 201 including the notch portions 13a and 15a is close to the characteristic change section 203 including the notch portions 13a and 15a in the circumferential direction of the optical sensor 10, however the non-superimposing portions 25a are arranged. Thus, to assure the strength of the optical sensor 10 in the portions where these members are arranged, the notch portions 13a and 15a are easily processed, and the characteristic change sections 201 and 203 are easily arranged in the notch portions 13a and 15a.

Furthermore, since the non-superimposing portions 25a are arranged, a length of the superimposing portion 25b shown in FIG. 1 is shorter than a length of the superimposing portion 25b depicted in the FIG. 4A. Thus, a length of the part 15b depicted in FIG. 1 is shorter than a length of the part 15b shown in FIG. 4A. Thus, when the optical sensor 10 bends, the part 15b of the protection member 15 is prevented from shifting from the light confinement member 13 in, e.g., the circumferential direction of the optical sensor 10, and also prevented from coming-off.

Moreover, the characteristic change section 201 is arranged to shift from the characteristic change section 203 in the circumferential direction of the optical sensor 10. Thus, bending directions of the optical sensor 10 itself are detected, and bending amounts in the bending directions of the optical sensor 10 itself are detected. At this time, in this embodiment, two axes are easily detected by the single optical sensor 10.

This detection will now be briefly described hereinafter.

The light source 101 emits light. The light enters the supply light guide member 103, and is guided to the optical branch section 107 by the supply light guide member 103. At this time, the light is branched to the optical sensor 10 by the optical branch section 107. Moreover, the light enters the light guide member 11 of the optical sensor 10, and is guided by the light guide member 11.

At this time, in a part of the light, the optical characteristics of the light are changed by the characteristic change sections 20. A change of optical characteristics corresponds to, e.g., a bending amount of the bending section 313 in which the optical sensor 10 is arranged. It is to be noted that, for example, when the inserting section 310 is inserted into a lumen, the bending section 313 bends in the lumen. The part of the light having the changed optical characteristics and other parts of the light having the unchanged optical characteristics are guided to the reflecting section 25c by the light guide member 11, and reflected by the reflecting section 25c. Additionally, in the part of the reflected light, the optical characteristics of the light are again changed by the characteristic change sections 20. As described above, the optical characteristics of the light are changed twice by the characteristic change sections 20. The part of the light having the changed optical characteristics and the other parts of the light having the unchanged optical characteristics are guided to the optical branch section 107 by the light guide member 11. At this time, the light is branched to the detecting section 105 by the optical branch section 107. Additionally, the light enters the detecting section 105.

The change in optical characteristics will now be briefly described hereinafter.

The optical characteristics represent optical absorption characteristics shown in FIG. 2B or wavelength conversion characteristics shown in FIG. 2C. A description will now be given as to an example where the characteristic change sections 201 and 203 have the optical absorption characteristics and the light travels from the optical branch section 107 to the reflecting section 25c.

An amount of the light absorbed by the characteristic change section 201 differs depending on a bending amount of the bending section 313, which is a bending amount of the optical sensor 10 arranged in the bending section 313 in particular.

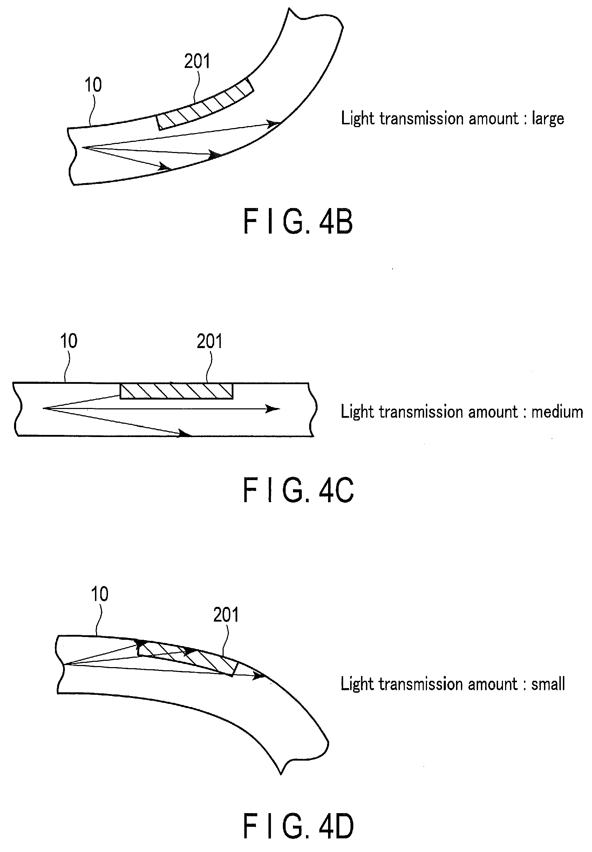

For example, when the optical sensor 10 bends upwards so that the characteristic change section 201 is placed on the inner side of the bending optical sensor 10 as shown in FIG. 4B, an amount of the light absorbed by the characteristic change section 201 is reduced to be smaller than that when the optical sensor 10 is straight as shown in FIG. 4C. Thus, a transmission amount of the light (which will be referred to as a light transmission amount hereinafter) transmitted to the detecting section 105 increases.

Further, for example, when the optical sensor 10 bends downward so that the characteristic change section 201 is placed on the outer side of the bending optical sensor 10 as shown in FIG. 4D, an amount of the light absorbed by the characteristic change section 201 increases to be larger than that when the optical sensor 10 is straight as shown in FIG. 4C. Thus, the light transmission amount is reduced.

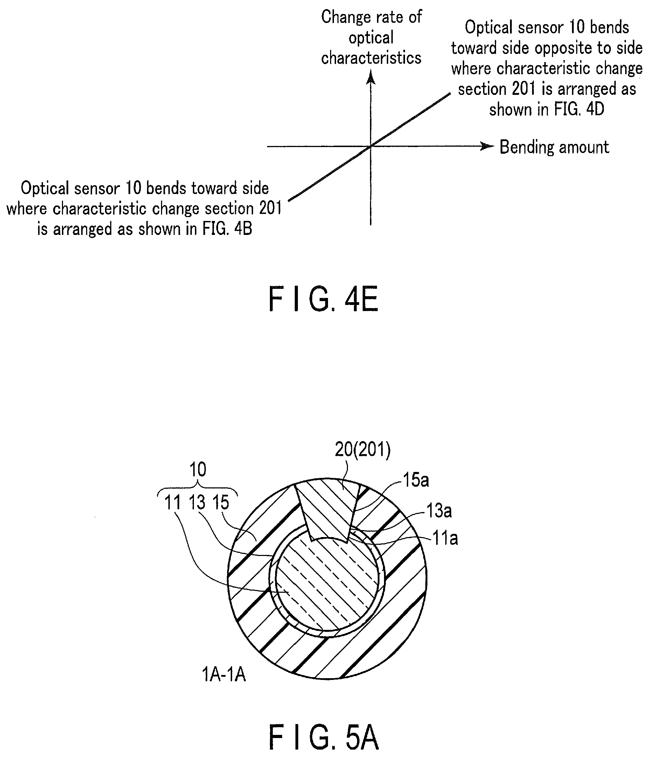

That is, as shown in FIG. 4E, a change rate of the optical characteristics in FIG. 4D is higher than a change rate of the optical characteristics in FIG. 4B.

When the amount of the light absorbed by the characteristic change section 20 increases or decreases, the amount of the light transmitted to the detecting section 105 varies. The transmission amount of the light transmitted to the detecting section 105 varies depending on the bending amount of the optical sensor 10, and it decreases as the bending amount increases. This relationship is shown in FIG. 4E.

The above-described contents also hold for the characteristic change section 203.

It is to be noted the above description also substantially holds when the light travels from the reflecting section 25c to the optical branch section 107.

It is to be noted that the optical absorption characteristics have been described above, but this is substantially applies to the wavelength conversion characteristics, and an amount of the light subjected to wavelength conversion by the characteristic change section 20 varies depending on a bending amount of the optical sensor 10.

As described above, the characteristics change sections 201 and 203 change the optical characteristics in accordance with, e.g., a bending amount of the optical sensor 10. Furthermore, the detecting section 105 independently detects the light having the optical characteristics changed by the characteristic change section 201 and the light having the optical characteristics changed by the characteristic change section 203. Moreover, the detecting section 105 detects a bending direction of the optical sensor 10 based on the independently detected optical characteristics A and B, and further detects a bending amount of the optical sensor 10 itself in the bending direction. Consequently, the bending amount and the bending direction of the bending section 313 where the optical sensor 10 is arranged are detected.

As to the bending amount, the bending amount in the up-and-down direction is detected by the characteristic change section 201, and the bending amount in the left-and-right direction is detected by the characteristic change section 203.

Additionally, as to the bending direction, the up-and-down direction is detected by the characteristic change section 201, and the left-and-right direction is detected by the characteristic change section 203.

As described above, the single optical sensor 10 can easily detect two axes.

### [Effects]

In this embodiment, the characteristic change section 201 is arranged at a position different from the characteristic change section 203 in the circumferential direction of the optical sensor 10. Further, as to the characteristic change sections 201 and 203 in this state, the characteristic change section 201 is arranged at a position different from the characteristic change section 203 in the axial direction of the optical sensor 10. Furthermore, the characteristic change sections 201 and 203 change the optical characteristics so that the optical characteristics A as one changed by the characteristic change section 201 become different from the optical characteristic B as the other changed by the characteristic change section 203.

Consequently, in this embodiment, the single optical sensor 10 can easily detect two axes, the notch portions 13a and 15a can be easily processed so that the strength of the optical sensor 10 can be assured, and the characteristic change sections 201 and 203 can be easily arranged in the notch portions 13a and 15a.

Moreover, in this embodiment, since the non-superimposing portion 25a is arranged, the protection member 15 can be prevented from shifting from the light confinement member 13 in, e.g., the circumferential direction of the optical sensor 10, and also prevented from coming off.

Additionally, in this embodiment, since the above operation can be carried out by using the single optical sensor 10, an arrangement space of the optical sensor 10 can be reduced. In this embodiment, the optical sensor 10 can be easily arranged even in a narrow tubular member like the bending section 313, thus readily carrying out the above operation.

Further, in this embodiment, positions of the characteristic change sections 20 can be easily identified from the appearance of the optical sensor 10. Consequently, in this embodiment, when the optical sensor 10 is arranged in a narrow tubular member like the bending section 313, the characteristic change sections 20 can be easily positioned to the tubular member.

It is to be noted that arrangement positions of the characteristic change sections 201 and 203 including the notch portions 13a and 15a are not restricted to the above-described positions as long as the light propagated through the light guide member 11 is not unnecessarily lost in the characteristic change sections 20 and the strength of the optical sensor 10 can be assured. This point will now be described hereinafter.

As shown in FIG. 5A, for example, the light guide member 11 may have a notch portion 11a which is arranged on the same straight line to the notch portion 13a in the radial direction of the optical sensor 10, communicates with the notch portion 13a in the radial direction of the optical sensor 10, and is formed by notching a part of the light guide member 11. Furthermore, the characteristic change sections 20 may be arranged in the notch portions 11a, 13a, and 15a to bite into the light guide member 11, respectively. FIG. 5A shows a cross-sectional position taken along 1A-1A depicted in FIG. 1.

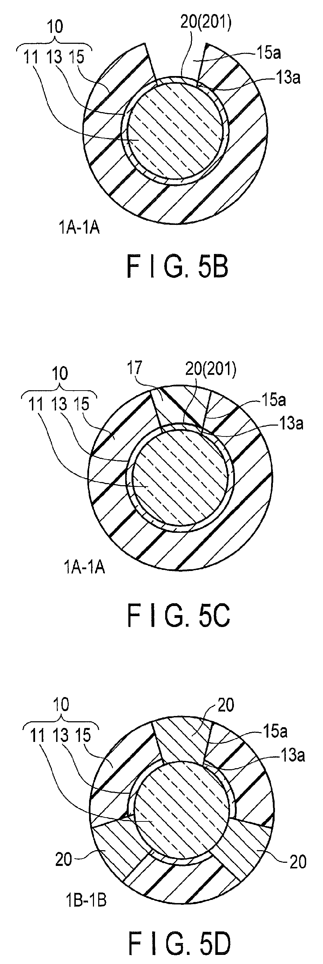

Moreover, as shown in FIG. 5B, the characteristic change section 20 may be arranged in the notch portion 13a of the light confinement member 13 alone. In this case, the member is not arranged in the notch portion 15a of the protection member 15 so that the outer peripheral surface of the characteristic change section 20 is exposed. Alternatively, as shown in FIG. 5C, a different member 17 may be embedded in notch portion 15a of the protection member 15. The different member 17 is a member that has the same material as the protection member 15 but is different from the protection member 15. Alternatively, although not shown, a member having the same refraction index as that of the light confinement member 13 or a member having a refraction index lower than that of the light confinement member 13 may be embedded in the notch portion 15a of the protection member 15. Each of FIG. 5B and FIG. 5C shows a cross-sectional position taken along 1A-1A depicted in FIG. 1.

Additionally, although the two characteristic change sections 20 are arranged, the present invention is not restricted thereto. As shown in FIG. 5D, three characteristic change sections 20 may be arranged, and they are arranged at intervals of, e.g., 120 degrees in the circumferential direction of the optical sensor 10. FIG. 5D shows a cross-sectional position taken along 1B-1B depicted in FIG. 1.

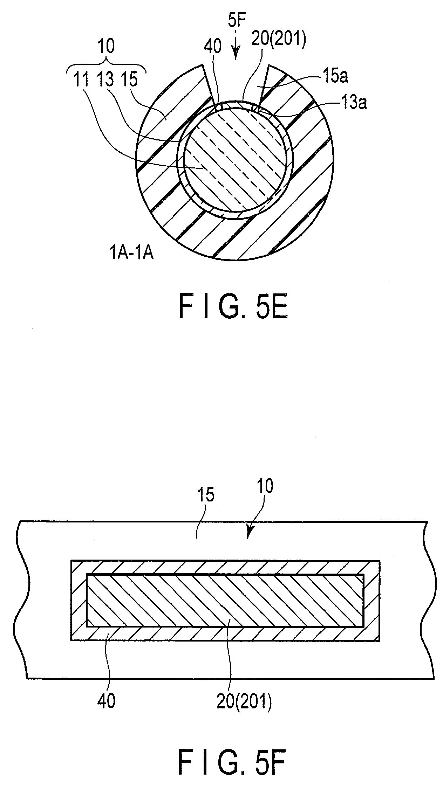

Further, as shown in FIG. 5E, the optical sensor 10 also has an inflow preventing section 40 that prevents the characteristic change section 20 from flowing into between the light guide member 11 and the light confinement member 13. Specifically, for example, the inflow preventing section 40 prevents the characteristic change section 20 from flowing into the notch portion 15a where the characteristic change section 203 is arranged from the notch portion 13a where the characteristic change section 201 is arranged. As shown in FIG. 5F, the inflow preventing section 40 is arranged in the notch portion 13a to surround the characteristic change section 20. The inflow preventing section 40 is made of a material having higher viscosity than that of the material of the characteristic change sections 20. This material has, e.g. , a refraction index that is substantially equal to that of the light confinement member 13, and has softness.

For example, a situation where the characteristic change section 201 flows into the distal end portion 203a from the proximal end portion 201b by a capillary phenomenon and is mixed with the characteristic change section 203. However, the inflow preventing section 40 prevents this inflow and also prevents mixture of the characteristic change section 201 and the characteristic change section 203.

It is to be noted that the inflow preventing section 40 is arranged in the notch portion 13a and prevents each characteristic change section 20 from flowing into the notch portion 15a where the characteristic change section 203 is arranged from the notch portion 13a where the characteristic change section 201 is arranged. However, the present invention is not restricted thereto, and the inflow preventing section 40 may be arranged in the notch portion 15a and may prevent each characteristic change section 20 from flowing into the notch portion 13a where the characteristic change section 201 is arranged from the notch portion 15 where the characteristic change section 203 is arranged.

Further, in this embodiment, the single area 30 is arranged in the optical sensor 10, but the present invention does not have to be restricted thereto.

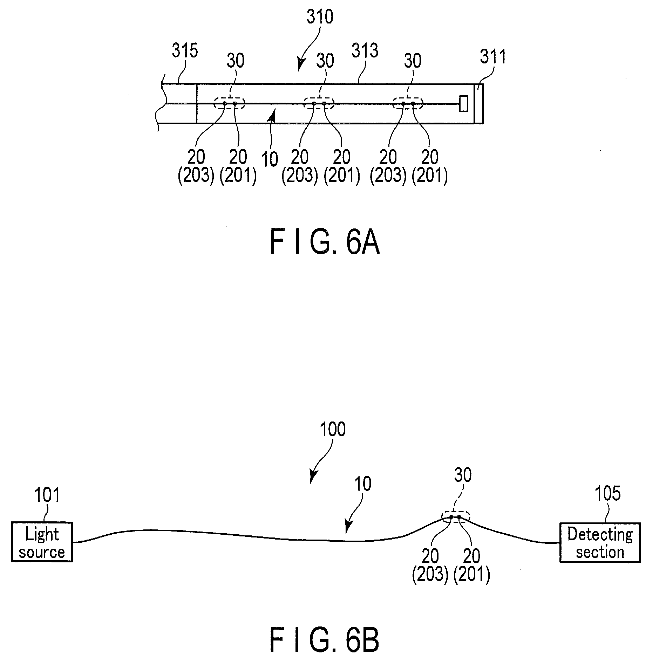

As shown in FIG. 6A, the multiple areas 30 may be arranged. These areas 30 are arranged apart from each other in the axial direction of the optical sensor 10. For example, the areas 30 are arranged at equal intervals from the distal end portion of the bending section 313 to the proximal end portion of the bending section 313. Consequently, in this embodiment, for example, bending directions of the bending section 313 and bending amounts of the bending section 313 can be variously detected in the axial direction of the bending section 313.

On the other hand, when the characteristics of the characteristic change section 20 in one area 30 are equal to the characteristics of the characteristic change section 20 in the other area 30, a uniformed bending value may be able to be detected over a wide range.

Furthermore, when the characteristics of the characteristic change section 20 in one area 30 are different from the characteristic of the characteristic change section 20 in the other area 30, multiple bending values may be able to be detected.

Moreover, in this embodiment, since the optical sensor system 100 is arranged in the endoscope 300, such a configuration as shown in FIG. 3A is provided. However, the configuration of the optical sensor system 100 is not restricted thereto.

As shown in FIG. 6B, the optical sensor system 100 may have the light source 101, the optical sensor 10, and the detecting section 105. The light source 101 is arranged at one end portion of the optical sensor 10, and the detecting section 105 is arranged at the other end portion of the optical sensor 10. As described above, the configuration of the optical sensor system 100 may be simplified in accordance with devices mounted therein.

Additionally, the optical sensor 10 in the optical sensor system 100 could be arranged in a non-illustrated small precise device. This small precise device is a tubular elongated member having flexibility, e.g., the inserting section 310 of the medial endoscope 300, an inserting section of an industrial endoscope, a manipulator, or a catheter.

### [Modifications]

In the first embodiment, the characteristic change sections 201 and 203 are arranged, and the non-superimposing portions 25a and the superimposing portion 25b are arranged. However, the present invention does not have to be restricted thereto. This point will be described hereinafter as a modification. A description will be given as to structures different from the structures of the first embodiment alone hereinafter. It is to be noted that like reference numerals denote structures equal to those in the first embodiment to omit a detailed description thereof.

### [First Modification]

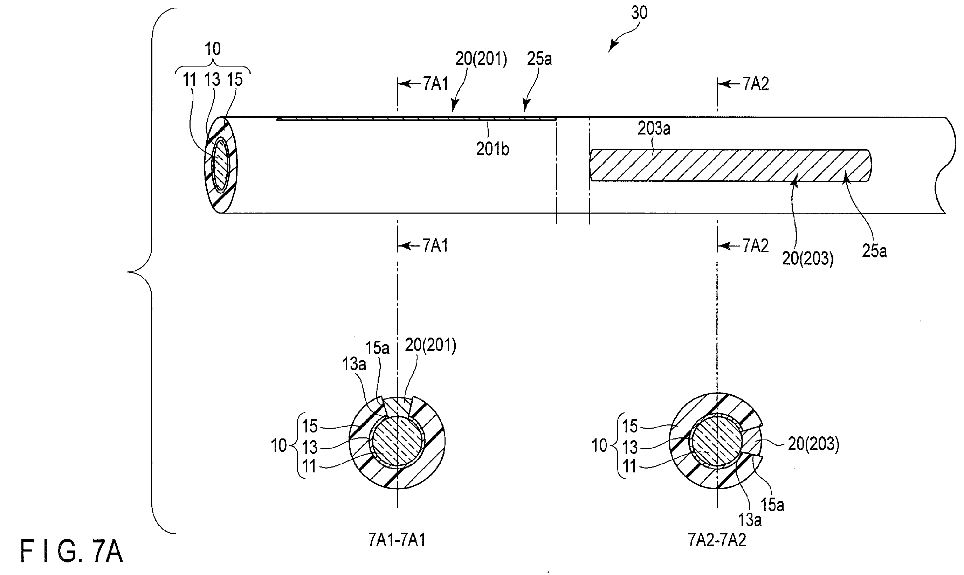

As shown in FIG. 7A, an overall characteristic change section 201 is arranged at a position different from a position of an overall characteristic change section 203 in an axial direction of an optical sensor 10 and a circumferential direction of the optical sensor 10. For example, the overall characteristic change section 201 is arranged to be closer to a distal end of the optical sensor 10 than the overall characteristic change section 203 in the axial direction of the optical sensor 10. That is, the proximal end portion 201b of the characteristic change section 201 is arranged to be closer to the distal end of the optical sensor 10 than the distal end portion 203a of the characteristic change section 203 in the axial direction of the optical sensor 10. Consequently, the proximal end portion 201b of the characteristic change section 201 is arranged on the distal end side of the optical sensor 10 at a desired interval from the distal end portion 203a of the characteristic change section 203 in the axial direction of the optical sensor 10.

Thus, the overall characteristic change section 201 is arranged not to be superimposed on the overall characteristic change section 203 in the circumferential direction of the optical sensor 10. That is, the overall characteristic change section 201 is arranged not to be on the same circumference to the overall characteristic change section 203 in the circumferential direction of the optical sensor 10.

Thus, in this modification, the overall characteristic change sections 201 and 203 function as the non-superimposing portions 25a, and the optical sensor 10 has the non-superimposing portions 25a alone. Further, when this modification is combined with the first embodiment, at least a part of the characteristic change section 201 is arranged at a position different from at least a part of the characteristic change section 203 in the axial direction and the circumferential direction of the optical sensor 10.

In this modification, the overall characteristic change section 201 is arranged at a position different from the overall characteristic change section 203 in the axial direction and the circumferential direction of the optical sensor 10. Thus, in this modification, notch portions 13a and 15a where the characteristic change sections 201 and 203 are arranged can be easily and rapidly processed, and the notch portions 13a and 15a that are apart from each other by 90 degrees in the circumferential direction of the optical sensor 10 can be easily processed. Furthermore, the strength of the optical sensor 10 can be readily assured.

### [Second Modification]

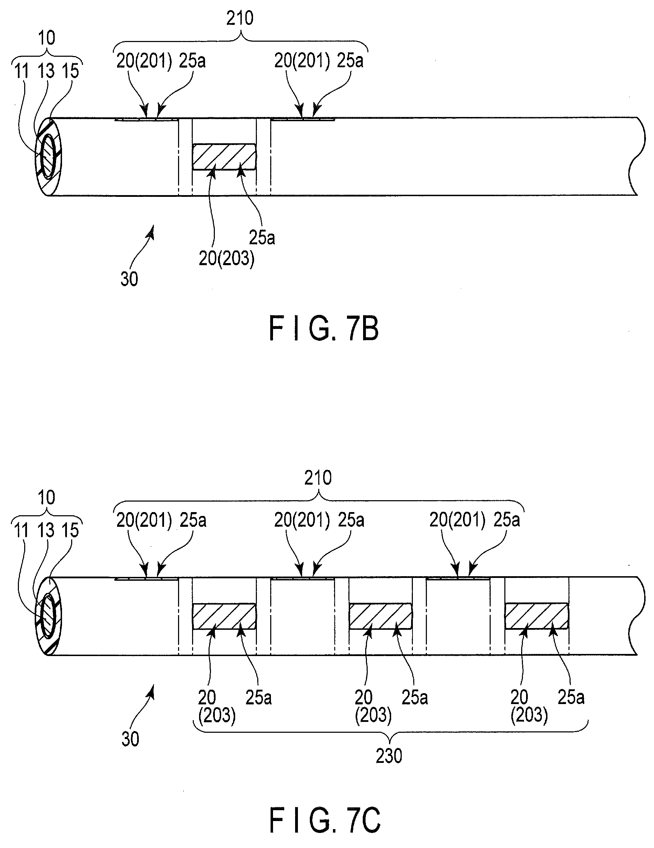

As shown in FIG. 7B, at least either multiple characteristic change sections 201 or multiple characteristic change sections 203 are provided, and form one group. A description will now be given as to an example where the multiple characteristic change sections 201 are arranged and the characteristic change sections 201 form a group 210 hereinafter.

For example, two characteristic change sections 201 are arranged. The characteristic change sections 201 are coaxially arranged.

For example, one characteristic change section 203 is arranged. The characteristic change section 203 is arranged at a position different from the characteristic change sections 201 in the circumferential direction of an optical sensor 10. The characteristic change section 203 is arranged between the characteristic change sections 201 in the axial direction of the optical sensor 10. The overall characteristic change section 203 is arranged not to be superimposed on the overall characteristic change sections 201 in the circumferential direction of the optical sensor 10. That is, the overall characteristic change section 203 is arranged not to be on the same circumference as the overall characteristic change sections 201 in the circumferential direction of the optical sensor 10.

Thus, the characteristic change sections 201 and 203 function as non-superimposing portions 25a, and the optical sensor 10 has the non-superimposing portions 25a alone.

In this modification, since the two characteristic change sections 201 are arranged, a bending amount and a bending direction of the optical sensor 10 on the characteristic change section 201 side can be more precisely detected. Moreover, in this modification, since the overall characteristic change section 203 is arranged not to be superimposed on the overall characteristic change sections 201 in the circumferential direction of the optical sensor 10, the above-described configuration can be carried out in a state that the strength of the optical sensor 10 is readily assured.

### [Third Modification]

As shown in FIG.7C, in this modification, multiple characteristic change sections 201 form a group 210, and multiple characteristic change sections 203 form a group 230. The number of the characteristic change sections 201 in the group 210 is equal to the number of the characteristic change sections 203 in the characteristic change section 203.

For example, three characteristic change sections 201 are arranged. The characteristic change sections 201 are coaxially arranged, and also arranged in rows.

For example, three characteristic change sections 203 are arranged which are equal to the characteristic change section 201 in number. The characteristic change sections 203 are coaxially arranged, and also arranged in rows.

A length of the characteristic change section 201 in this modification is shorter than a length of the characteristic change section 201 in the first embodiment. This point is likewise applied to the characteristic change section 203.

The group 210 is arranged at a position different from the group 230 in both the circumferential direction and an axial direction of an optical sensor 10. In other words, the group 210 is arranged to shift from the group 230 in the circumferential direction and the axial direction of the optical sensor 10. Specifically, one overall characteristic change section 201 is arranged at a position different from one overall characteristic change section 203 in the circumferential direction and the axial direction of the optical sensor 10.

Thus, in this modification, the whole groups 210 and 230 function as non-superimposing portions 25a, and the optical sensor 10 has the non-superimposing portions 25a alone.

Further, the characteristic change sections 201 in the group 210 and the characteristic change sections 203 in the characteristic change section 203 are alternately arranged in the axial direction of the optical sensor 10.

In this modification, in one area 30, the multiple characteristic change sections 201 and the multiple characteristic change sections 203 are arranged. Thus, in this modification, as compared with a state where one characteristic change section 201 and one characteristic change section 203 are arranged, a bending amount and a bending direction of the optical sensor 10 can be further precisely and averagely detected.

### [Fourth Modification]

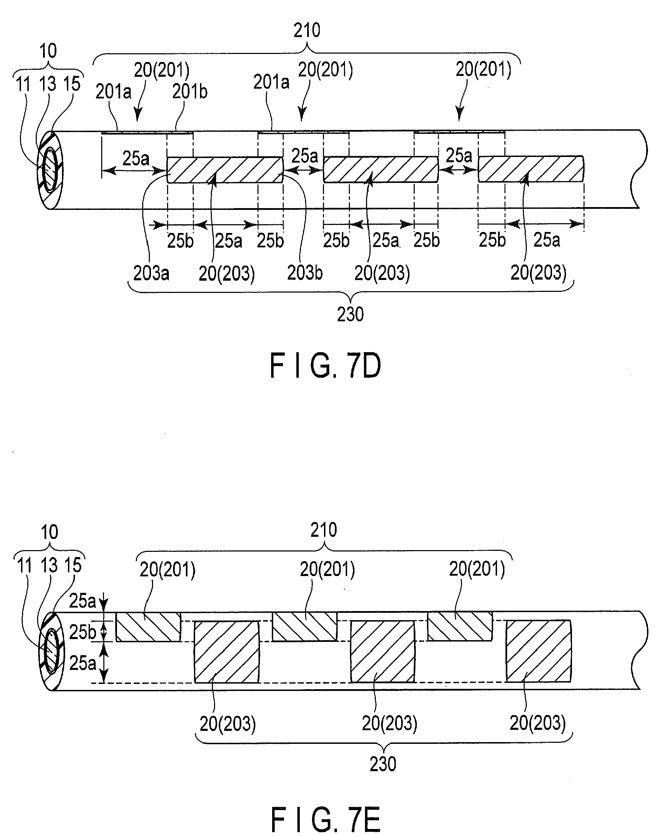

As shown in FIG. 7D, arrangement positions of groups 210 and 230 and the numbers of characteristic change sections 201 and 203 in this modification are substantially equal to those in the third modification.

In this modification, like the first embodiment, differing from the third modification, an optical sensor 10 has superimposing portions 25b and non-superimposing portions 25a sandwiching each superimposing portion 25b in an axial direction of the optical sensor 10. This point will now be described hereinafter.

For example, as regards one characteristic change section 201 and the characteristic change section 203 which is arranged to be adjacent to this characteristic change section 201 in the axial direction of the optical sensor 10 and arranged to be closer to the proximal end portion side of the optical sensor 10 than this characteristic change section 201, the following arrangement is carried out.

Like the first embodiment, the distal end portion 201a of the characteristic change section 201 is arranged not to be superimposed on the distal end portion 203a of the characteristic change section 203 in the circumferential direction of the optical sensor 10. The non-superimposition includes non-overlapping. In other words, the distal end portion 201a of the characteristic change section 201 is arranged not to be on the same circumference of the optical sensor 10 to the distal end portion 203a of the characteristic change section 203.

Like the first embodiment, the proximal end portion 201b of the characteristic change section 201 is arranged to be superimposed on the distal end portion 203a of the characteristic change section 203 in the circumferential direction of the optical sensor 10. The superimposition incudes overlapping. In other words, the proximal end portion 201b of the characteristic change section 201 is arranged to be on the same circumference of the optical sensor 10 to the distal end portion 203a of the characteristic change section 203, and aligned with the same. As described above, the proximal end portion 201b is arranged at the same position to the distal end portion 203a in the axial direction of the optical sensor 10, but is arranged at a position different from the proximal end portion 203b in the circumferential direction of the optical sensor 10.

Furthermore, as regards the characteristic change section 203 and the characteristic change section 201 that is arranged to be adjacent to this characteristic change section 203 in the axial direction of the optical sensor 10 and arranged to be closer to the proximal end portion side of the optical sensor 10 than this characteristic change section 203, the following arrangement is carried out.

The distal end portion 201a of the characteristic change section 201 is arranged to be superimposed on a proximal end portion 203b of the characteristic change section 203 in the circumferential direction of the optical sensor 10. The superimposition includes overlapping. In other words, the distal end portion 201a of the characteristic change section 201 is arranged to be on the same circumference of the optical sensor 10 to the proximal end portion 203b of the characteristic change section, and aligned with the same. As described above, although the distal end portion 201a is arranged at the same position to the proximal end portion 203b in the axial direction of the optical sensor 10, it is arranged at a position different from the proximal end portion 203b in the circumferential direction of the optical sensor 10.

With the above-described configuration, a length of the characteristic change section 201 in this modification can be increased beyond the length of the characteristic change section 201 in the third modification. This point can be likewise applied to the characteristic change section 203.

As described above, a part (e.g. , the proximal end portion 201b) of each characteristic change section 201 in the group 210 is arranged at the same position to a part (e.g. , the distal end portion 203a) of each characteristic change section 203 in the group 230 in the axial direction of the optical sensor 10, but is arranged at a different position in the circumferential direction of the optical sensor 10.

In this modification, since the characteristic change sections 201 and the characteristic change sections 203 are arranged in one area 30, a bending amount and a bending direction of the optical sensor 10 can be further precisely and averagely detected as compared with a state where one characteristic change section 201 and one characteristic change section 203 are arranged.

### [Fifth Modification]

As shown in FIG. 7E, a thickness of each characteristic change section 201 in this embodiment is larger than a thickness of the characteristic change section 201 in the first embodiment. This point can be likewise applied to the characteristic change sections 203.

Thus, a part of each characteristic change section 201 is arranged on the same straight line as a part of each characteristic change section 203 in an axial direction of an optical sensor 10. These parts represent end portions of the characteristic change sections 201 and 203 in a circumferential direction of the optical sensor 10, respectively. In other words, a part of the characteristic change section 203 is arranged between a part of one characteristic change section 201 and a part of the other characteristic change section 203 in the axial direction of the optical sensor 10.

Thus, the optical sensor 10 has a superimposing portion 25b and non-superimposing portions 25a that sandwich the superimposing portion 25b therebetween in the circumferential direction of the optical sensor 10.
As described above, a part of each characteristic change section 201 in the group 210 is arranged at a position different from a part of each characteristic change section 203 in the group 230 in the axial direction of the optical sensor 10, but is arranged at the same position in the circumferential direction of the optical sensor 10.

In this modification, since the characteristic change sections 201 and 203 according to this modification are thicker than the characteristic change sections 201 and 203 according to the first embodiment and they can contribute to, e.g., optical absorption, a bending amount and a bending direction of the optical sensor 10 can be further precisely and averagely detected with high sensitivity.

### [Conclusion]

When the third modification to the fifth modification are carried out, a part of each characteristic change section 201 in the group 210 is to be arranged at a position different from a part of each characteristic change section 203 in the group 230 in at least one of the circumferential direction of the optical sensor 10 and the axial direction of the optical sensor 10.

Moreover, the present invention is not restricted to the foregoing embodiment as it is, and can be embodied by modifying constituent elements without departing from a gist thereof in an embodying stage. Additionally, appropriately combining the constituent elements disclosed in the foregoing embodiment can lead to formation of various inventions.

## Claims

1. An optical sensor comprising: a light guide member which guides light; a light confinement member which covers an outer peripheral surface of the light guide member to abut on the outer peripheral surface of the light guide member, and confines the light in the light guide member; a protection member which covers an outer peripheral surface of the light confinement member to abut on the outer peripheral surface of the light confinement member, and protects the light confinement member; and characteristic change sections which are in contact with at least the light guide member, and change optical characteristics of the light in accordance with a bending amount of the light guide member,
wherein one characteristic change section and the other characteristic change section are arranged to be close to each other in an area having a desired range,
at least a part of the one characteristic change section is arranged at a position different from at least a part of the other characteristic change section in a circumferential direction of the optical sensor,
at least a part of the one characteristic change section is arranged at a position different from at least a part of the other characteristic change section in an axial direction of the optical sensor,
the one characteristic change section changes the optical characteristics in accordance with the bending amount in one direction of the light guide member in the area,
the other characteristic change section changes the optical characteristics in accordance with the bending amount in the other direction of the light guide member in the area, and
the characteristic change sections change the optical characteristics so that the one optical characteristics changed by the one characteristic change section are different from the other optical characteristics changed by the other characteristic change section, when the optical characteristics of the light change.

2. The optical sensor according to claim 1,
wherein the one overall characteristic change section is arranged at a position different from the other overall characteristic change section in the axial direction of the optical sensor and the circumferential direction of the optical sensor.

3. The optical sensor according to claim 2,
wherein at least one of the one characteristic change section and the other characteristic change section is arranged in a plural number to form one group.

4. The optical sensor according to claim 3,
wherein the characteristic change sections on one side form a first group,
the characteristic change sections on the other side form a second group, and
the characteristic change sections on one side in the first group and the characteristic change sections on the other side in the second group are alternately arranged in the axial direction of the optical sensor.

5. The optical sensor according to claim 4,
wherein the number of the characteristic change sections on the one side in the first group is equal to the number of the characteristic change sections on the other side in the second group.

6. The optical sensor according to claim 5,
wherein a part of each of the characteristic change sections on the one side in the first group is arranged at a position different from a part of each of the characteristic change sections in the second group in at least one of the circumferential direction of the optical sensor and the axial direction of the optical sensor.

7. The optical sensor according to claim 6,
wherein the characteristic change sections on the one side in the first group are arranged at positions different from the characteristic change sections on the other side in the second group in both the circumferential direction of the optical sensor and the axial direction of the optical sensor.

8. The optical sensor according to claim 3,
wherein the one direction is orthogonal to the other direction.

9. The optical sensor according to claim 1, further comprising an inflow preventing section which prevents the characteristic change section from flowing into between the light guide member and the light confinement member.

10. The optical sensor according to claim 1,
wherein the area is provided in a plural number, and
the areas are arranged apart from each other in the axial direction of the optical sensor.

11. An optical sensor system comprising:
a light source which emits the light toward the light guide member;
the optical sensor according to claim 1; and
a detecting section which independently detects the light having the optical characteristics on the one side and the light having the optical characteristics on the other side, and detects mutually different bending directions each other and mutually different bending amounts each other in the optical sensor based on a detection result.

12. An endoscope comprising the optical sensor system according to claim 11.
